# EUROPEAN PATENT SPECIFICATION

(11) **EP 1 961 402 B1**
(45) Date of publication and mention of the grant of the patent: **05.01.2011**
(21) Application number: 07123747.3
(22) Date of filing: 20.12.2007
(51) Int. Cl.: A61F 13/15

(54) **Absorbent article with barrier sheet**
Saugfähiger Artikel mit Barriereschicht
Article absorbant avec feuille barrière

(30) Priority: 13.02.2007 EP 07102195; 13.02.2007 EP 07102196
(43) Date of publication of application: 27.08.2008
(73) Proprietor: The Procter and Gamble Company, Cincinnati, OH 45202 (US)
(72) Inventor: Ponomarenko, Ekaterina Anatolyevna, 65812, Bad Soden (DE); Li, Wenbin, Mason, OH 45040 (US)
(74) Representative: McGregor, Judit Ester

(56) References cited:
- EP-A- 1 520 569
- WO-A-2005/004769
- WO-A-2005/103355
- US-A1- 2006 014 460
- US-A1- 2006 058 765
- US-A1- 2006 189 956

## Description

### FIELD OF THE INVENTION

This invention is directed to an absorbent article having a backsheet, an absorbent core and cuffs and/ or a topsheet, said topsheet being provided with at least one opening adapted to receive fecal material (also called anal/genital cuff), said cuffs or topsheet comprising a nonwoven sheet material comprising at least two nonwoven layers or webs, whereby said layers being typically only partially attached to one another, whereof the skin-contacting nonwoven layer or web has a specific low bending rigidity, whilst the nonwoven sheet as a whole has a very high barrier function.

### BACKGROUND OF THE INVENTION

It is well known that fecal material is often difficult to remove from the skin of the user, in particular on sensitive skin such as by young babies and the skin around the genitals. Moreover, it is well known that fecal material on the skin can cause irritation and redness of the skin and some times even dermatitis of the skin.

One of the solutions to reduce the fecal material on the skin is to provide a means to isolate the fecal material immediately after discharge, away from the skin. For example, diapers with a topsheet with an opening, also referred to as anal and/ or genital cuff, through which the feces can pass to a void space between the topsheet and the absorbent core, have been developed. The fecal material is then stored underneath this topsheet, away from the skin.

It has been found that it is desirable that the feces, once received through the opening, is not visible for the person changing the article, i.e. that the topsheet masks the feces. This is for example described in co-pending application EP-A-1417945, which describes hydrophobic topsheets that have a very high alcohol repellency, in order to provide no rewetting of the topsheet by the stored feces.

The inventors have now found that often materials that provide a good barrier are not very comfortable in use, because these materials may be too thick or too stiff. The inventors found that it may be desirable that the topsheet, also referred to as anal and/ or genital cuff, but also other cuffs, like barrier cuffs and leg cuffs, are made of a material that not only provides an excellent barrier for the feces, to avoid or reduce rewet of the skin by the stored feces, but that also, at the same time, is very soft and flexible for the sensitive (baby) skin. The inventors found a new means to provide both these properties at the same time, and in particular, in one embodiment herein, they found that nonwoven materials formed from two or more thin layers, whereof the skin contacting layer has a specific softness, whilst the other layer(s), or the material as a whole provides the required barrier properties, results in the improved performance, as described above.

### SUMMARY OF THE INVENTION

The present invention relates to an absorbent article comprising a topsheet, having typically one or more openings to receive bodily exudates, said topsheet comprising a nonwoven sheet, comprising two or more nonwoven layers or nonwoven webs, whereby the nonwoven sheet comprises two or more nonwoven layers, two of which are only partially attached, i.e. not fully laminated, to one another, with an attachment area of 60% or less, or 40% or less (as defined herein); and/ or preferably said two or more nonwoven layers each comprise two or more nonwoven webs that are laminated together (and said two or more nonwoven laminate layers being only partially attached to one another, as above); each comprising two or more nonwoven webs that are laminated to one another, and whereby the nonwoven sheet comprises nano-fibers and/ or meltblown fibers, said meltblown fibers preferably being present at a level of at least 5 g/m², and the nonwoven sheet having a basis weight of 40 g/m² or less, or preferably 30 g/m² or less.

It has been found that excellent barrier properties and softness and flexibility may be obtained if the two (or more) nonwoven layers are only partially attached, as described herein, and not fully laminated.

The invention also relates to an absorbent article comprising one or more leg and/ or barrier cuffs that comprise a nonwoven sheet, comprising two or more nonwoven layers or webs, typically as above, whereof at least one nonwoven layer or web, has a bending rigidity of 16 grams or less, preferably 12 grams or less, and said nonwoven sheet having a hydrostatic head (with 49 mN/m liquid- measured with the method set out herein) of at least 18 mbar, preferably at least 20 mbar (or at least 25 mbar, or at least 30 mbar), whereby said two or more nonwoven layers preferably comprise two or more laminated nonwoven webs, and said layers are only partially attached, i.e. not fully laminated, to one another, e.g. having an attachment area of 60% or less, or 40% or less.

### BRIEF DESCRIPTION OF THE FIGURES

Figure 1 shows a perspective view of a preferred absorbent article of the present invention, comprising a preferred topsheet or anal/ genital cuff.
Figure 2 shows a top view of the article of Figure 1.

### DETAILED DESCRIPTION OF THE INVENTION

As used herein, the following terms have the following meanings:

As used herein, 'absorbent article' means any article that can absorb body fluids and is suitable to be placed in close proximity to the genitals and/ or anus of the user (including in particular and adult, baby or infant diaper, including so-called training or pull-up pants).

As used herein, the "topsheet" is any unitary sheet or a multitude of (attached) sheets that are present over (part of) the absorbent core or (part of) the backsheet and facing or in contact with the skin of the user in use, and it includes so-called anal and/ or genital cuffs.

As used herein 'front region' and 'back region' refer to the two regions, which are in use, respectively, closest to the front of the wearer and the back of the wearer.

As used herein, the term 'void space' is a cavity in the article present in at least the relaxed state, which serves to accept and contain bodily exudates such as fecal material, for example having a volume of at least 3 or even 5 cm³ in relaxed state. When used herein, 'longitudinal' is the direction running substantially parallel to the maximum linear dimension of the component or article, and includes directions within 30° of this parallel, when applicable.

The 'lateral' or 'transverse' direction is perpendicular to said longitudinal direction and in the same plan of the majority of the article and the longitudinal axis.

As used herein 'stretched' or stretched state' means that the article or elasticated topsheet or cuff thereof is stretched to its maximum length.

As used herein 'relaxed' or 'relaxed state' means the state that no forces are applied to the article or elasticated topsheet or cuff thereof (other than naturally occurring forces such as gravity), when the article is laid on a horizontal surface on its backsheet, such that the transverse front and back edge are flat on the horizontal surface and the transverse centre line or axis is on the horizontal surface.

As used herein, 'elasticated' means typically, that the component comprises elastic material, which is elastic in at least one direction.

As used herein, 'along' means 'at least partially substantially parallel to and adjacent to'. Adjacent includes 'in close proximity with' and `in contact with'.

As used herein, 'opening' (as present in the topsheet or anal/ genital cuff) means an area circumscribed by the topsheet, but where the topsheet material is not present, and which is large enough to receive fecal material, for example being at least 2 cm long or wide, or having a surface area of at least 2 cm².

As used herein, a "nonwoven web" is a single web, whilst a "nonwoven layer" may comprise a multitude of nonwoven webs, and a "nonwoven sheet" may comprise a multitude of nonwoven layers.

In a first embodiment, the absorbent article of the invention comprises a topsheet (2) or with one or more openings, preferably a single opening, for the reception of fecal material. Preferably, the opening is in the form of a slit opening. The opening is preferably present in (part of) the front region (20) of the topsheet (in use towards the front of the user) and in (part of) the back region (22) of the topsheet. Preferably, the topsheet has a slit opening, which has a longitudinal dimension (length) substantially parallel to the longitudinal axis of the topsheet and of the diaper. Preferred is that (in stretched state) the opening (or openings) of the topsheet is (are) configured such that from 20% to 40%, or more preferably from 20% to 30% of the length of the opening (or total length of the openings) extends from the transverse axis of the topsheet towards the front edge of the topsheet, and the remaining percentage extends towards the back edge of the topsheet.

The dimensions and exact shape of the opening(s) may vary, depending on the size of the topsheet (2) and/ or the absorbent article. For example, in a preferred embodiment the opening is in the form of a slit opening with substantially parallel longitudinal side edges, which are connected in the front and back by V-shaped or rounded V-shaped (as shown herein after) front and back edges, wherein both the front and back V-shaped edges comprise two angled edges. The maximum length of the slit opening (in fully stretched state) may be for example 40% to 90% or more preferably 50% to 80%, or even more preferably about 60% to 70%, of the total length L of the absorbent article. The average width of the opening herein, in 75% stretched state, is preferably from 5% to 30%, or more preferably 10% to 25%, of the average width of the topsheet (including opening width), or for example for a size 4 diaper, 15 mm to 60 mm, more preferably from 20 mm to 40mm.

The topsheet of the absorbent article of the invention comprises a nonwoven sheet material with a skin-facing surface and a skin-facing nonwoven layer or web, i.e. that in use faces or is even (at least partially) in contact with the skin. The topsheet may comprise said nonwoven sheet over its whole surface area or part thereof (but for example at least 30% of the surface area that is in contact with the skin of the user, or of the total surface area of the topsheet comprises or consists of said nonwoven sheet described herein).

The invention also provides an absorbent article with one or more cuffs, e.g. leg cuffs (for example 30) and/ or barrier cuffs, that comprise a nonwoven sheet material as described herein, for example such that at least 40% of the surface area of the cuff comprises or consists of said nonwoven sheet material. Typically, the absorbent article comprises two cuffs that extend in longitudinal direction along the longitudinal side edges of the article, or part thereof, and that are typically attached to said article with one longitudinal edge of said cuff, thus having a free longitudinal edge that can be positioned out of the X-Y plane (longitudinal/ transverse directions) of the article, i.e. in z-direction. The cuffs are typically mirror images of one another in the Y-axis of the article.

In one embodiment, the topsheet or cuff herein comprises a nonwoven sheet that is a barrier sheet, said topsheet or cuff and/ or said nonwoven sheet having typically a hydrostatic head value (measured with a 49 mN/m liquid with the hydrostatic head test set out herein) of at least 18 mbar, preferably at least 20 mbar, or at least 25 mbar, or at least 28 mbar, or at least 30 mbar, or optionally at least 35 mbar, and optionally less than 50 or less than 45 mbar. A nonwoven sheet, topsheet or cuff herein is considered to have the above hydrostatic head values if it has this value at any part of the material, excluding areas comprising elastic material or edges attached to another material: i.e. the measurement is done on a sample that does not comprise elastic material or edges attached to another material. In one embodiment, the nonwoven sheet or cuff or topsheet has a surface are free of elastics or edges of at least 2.5 cm x 2.5 cm.

The nonwoven sheet of the topsheet or cuffs herein comprises at least two nonwoven layers or nonwoven webs, preferably at least two nonwoven layers. In one embodiment, at least one nonwoven layer or web is a skin-facing nonwoven layer or web (i.e. that in use faces the skin of the user and may contact the skin of the user), said skin-facing nonwoven layer or web having for example a bending rigidity of 20 grams or less, preferably 16 grams or less, or even 14 grams or less or 12 grams or less, as measured with the handle-o-meter rigidity/ softness test set out herein. Alternatively, or in addition the nonwoven sheet as a whole may have a bending rigidity of less than 35 grams, or less than 30 grams or less than 25 grams or less than 20 grams or less than 18 grams.

A nonwoven sheet, nonwoven layer, topsheet or the cuff herein is considered to have the above bending rigidity values if it has this value at any part of the material, excluding areas comprising elastic material or edges attached to other materials (these latter should not be included in the test).

The bending rigidity as referred to herein, and measured with the method herein, is the rigidity of said nonwoven layer, nonwoven web or nonwoven sheet in any direction, unless specified otherwise.

In one embodiment, the nonwoven sheet comprises on its skin-facing surface a nonwoven web or a nonwoven layer that has on its skin-facing surface a nonwoven web, said nonwoven web comprising fibers with an average fiber direction, and said nonwoven layer or nonwoven sheet has a bending rigidity of the values specified above, in said fiber direction. Preferred skin-facing webs with fibers with an average fiber direction are spunbond webs. Thus, the nonwoven sheet herein has preferably on its skin-facing surface a nonwoven web, which may be part of a nonwoven layer, and that comprises fibers with an average direction, such as a spunbond web with the above bending rigidity in the fiber direction.

The average fiber direction may typically be the machine direction (MD) of the absorbent article.

The nonwoven sheet as a whole is preferably soft having a bending rigidity as described above.

The nonwoven sheet or topsheet has in one embodiment or a preferred embodiment a low surface tension strike through value, as determined by the method described herein, of at least 30 seconds, preferably at least 5 seconds, or even at least 60 seconds, and optionally less than 200 seconds, or less than 150 seconds or less than 100 seconds. A nonwoven sheet, topsheet cuff is considered to have the above low surface tension strike through values if it has this value at any part of the material, excluding areas comprising elastic material or edges being attached to other materials.

As mentioned above, the nonwoven sheet material comprises two or more nonwoven layers that are attached to one another, but preferably not fully (i.e. 100%) laminated to one another. In one embodiment, said two (or more) nonwoven layers have an attachment area of 60% or less, or 40% or less than 40% (of the total area of overlap between two neighboring nonwoven layers), or even 20% or less. In one embodiment, the nonwoven layers are attached to one another along the side edges of the overlap area, e.g. along the edges of each or one of the nonwoven webs (periphery) and optionally the area where elastic component(s) is present, and the nonwoven layer comprises areas, e.g. of at least 0.5 cm², where both webs are present but not attached to one another. In one embodiment, the nonwoven sheet is such that at least two layers thereof are only partially attached to one another and there is at least one area of 2.5 x 2.5 cm that is not attached (and does not comprise elastics or edges).

At least one nonwoven layer, or each nonwoven layer typically comprises two or more nonwoven webs that are laminated to one another, i.e. each nonwoven layer is a laminate nonwoven layer.

In one embodiment, the nonwoven sheet comprises nano-fibers that have an average diameter of 1.0 microns or less. Preferred may be that the nonwoven sheet comprises two or more nonwoven layers, whereof one or more or each comprise a nonwoven web that comprise such nano-fibers. The nonwoven sheet or layer thereof may for example comprise at least 2 g/m² of nano-fibers, or at least 3 g/m² or at least 5 g/m² of nano-fibers. The nano-fibers may have an average diameter of 0.8 microns or less, or 0.6 microns or less. The nano-fibers may be made by known melt fibrillation methods or melt film fibrillation methods, such as described in US6,315,806 and US6,695,992. Preferred nano-fiber webs and layers are described in co-pending application WO2005/103355.

In one embodiment, at least one nonwoven layer, or each nonwoven layer comprises a nonwoven web of meltblown fibers, typically present at a weight level of at least 5 g/ m² by weight of the nonwoven layer, or for example at least 5.7 g/ m², or at least 7 g/ m², but typically less than 20 or 15 g/m² by weight of the nonwoven layer.

The nonwoven sheet may have a basis weight of 45 g/ m² or less, or 40 g/m2 or less or 35 g/m² or less or 30 g/m² or less. Preferred may be that the nonwoven sheet comprises only two nonwoven layers, each comprising two or more nonwoven webs. Preferred may be that the basis weight of each of the nonwoven layers present in said nonwoven sheet is 24 g/m² or less, or 22 g/m² or less or 18 g/m² or less.

The nonwoven sheet may comprise a hydrophobic agent, such as a wax. The nonwoven sheet or one or more layer thereof may also comprise a barrier agent, also referred to as masking facilitating agent, as described below.

As mentioned above, preferred nonwoven sheet herein have on one surface, e.g. the skin-facing surface a spunbond nonwoven web. Preferred is that the nonwoven sheet comprises nonwoven layers comprising spunbond webs (S) and meltblown webs (M) and/ or nano-fiber webs (N), whereby it may be preferred that the outer surface of the nonwoven sheet is formed by a spunbond web.

Preferred are herein are: a nonwoven sheet comprising a 17 or 22 gsm (g/m²) SMMMS nonwoven layer attached to (but not laminated to) another 17 or 22 gsm SMMMS nonwoven layer (whereof for example the meltblown level of each layer is 5.7 or 7.3 gsm respectively), including a nonwoven sheet comprising 22 gsm SMMMS nonwoven layer, with for example 7.3gsm meltblown fibers, attached to 17 gsm SMMMS or SMMS nonwoven layer, comprising for example 5.7 gsm meltblown fibers; a nonwoven sheet comprising a 17 gsm or 22 gsm SMS or SNS nonwoven layer, attached to another 17 gsm or 22 gsm SNS or SMS nonwoven layer; a 17 or 22 gsm SMMS nonwoven layer attached to a 17 or 22 gsm SMMS or SMMMS nonwoven layer (comprising for example 3 gsm or 5.7 gsm (for SMMS) or 7.3 gsm meltblown fibers per layer).

The topsheet or cuffs herein preferably comprise one or more elastic components, as described herein below. The elastic component is preferably attached to the nonwoven sheet material herein, so that the topsheet, cuff and nonwoven sheet comprise elastic laminate portions, herein referred to also as elastic areas (e.g. the portions (31) of the topsheet (2)). Preferably, the elastic component is attached to a surface of the nonwoven sheet that in use is not facing the skin, or contacting the skin, and/ or it is attached between two of the nonwoven layers that may be present in the nonwoven sheet, and/ or it is attached to the nonwoven sheet and covered with a covering strip material, so the elastic component is not in direct contact with the skin of the user in use.

The elastic laminate portions (e.g. comprising the nonwoven sheet and elastic at least) may comprise a straight portion, that comprise wrinkles in (partially) contracted/ unstretched state, which are preferably very small wrinkles, e.g. said straight portion having an average wrinkle height, as measured by the method set out below, of less than 1000 microns, or less than 850 micron or less than 600 microns. To measure this, the straight elastic laminate portion is elongated (stretched) to the length that it has an elongation ε= 0.5 (e.g. if contracted length is 20 cm, the laminate portion is stretched to the partially stretched length of 30 cm). This laminate portion is examined by use of PRIMOS and its data acquisition software, following the manufacture's instructions manual, using a 13x18mm lens. This will calculate the average wrinkle height (and density) of the straight elastic laminate portion. (If the elastic laminate portion has an average width of more than 3 mm, then the measurement above is only done on the inner 70% of the width of the laminate portion, along its full length.)

Such an elasticated portion or area (31) in the topsheet (2) or cuff herein may be formed from a multitude of thin strands of elastic material or for example from a single band of elastic material. The cuffs may comprise such elasticated areas along part or all of the longitudinal edge, e.g. the free edge, of the cuff.

The topsheet may comprise an elasticated area or elastic laminate portion at least along or in close proximity to each (longitudinal) side edge of the opening or openings, described herein.

The elasticated area or areas of the topsheet extend preferably from said longitudinal side edge of the opening(s) towards or completely to the front and/ or back transverse edge of the topsheet. The elasticated areas are preferably longer than the opening. The width of the elasticated areas on the cuff or topsheet will vary, typically depending on the exact dimensions of the topsheet and/ or the article, for example the elasticated area may have an average width of about 1 mm to 40 mm, or 2 mm to 30 mm, or 2 mm, or even 3 mm to 20 mm. The elastic materials are typically in the form of a single strand, multitude of strands, with any thickness or width, or a single strand or band with an average thickness (e.g. gauge) of at least 20 microns, more preferably at least 40 microns, or even at least 60 microns, typically up to about 300 microns, or even up to 200 microns or even up to 150 microns. Useable elastic materials used hereto include Lycra, VFE-CD, available from Tredegar, and L-86, L-89, or L-90, available from Fulflex (Limerick, Ireland).

The topsheet preferably comprises at least two elasticated areas or portions, each along part or all of a longitudinally extending side edge of the opening or openings, said elastic areas or portions, and preferably said side edges, being mirror images of one another in the y-axis of the topsheet or article.

The front end portions of two opposing elasticated areas may bend away from one another (in the plane of the topsheet), so that the distance between the end edges of the opposing front end portions of two opposing elasticated areas is larger that the distance between the two longitudinal centre points of two opposing elastic areas, and equally, the distance between the end edges of the opposing back end portions of two opposing elasticated areas may be larger that the distance between the longitudinal centre points of two opposing elastic area.

The front end portion of an elasticated area may have an angle with a longitudinal line through the centre point of the elasticated area and parallel to the longitudinal axis of the topsheet, said angle being between 10° and 40°, or preferably between 17° to 35°, or even more preferably between 20° and 35°.

The back end portion of an elasticated area may also have an angle with a longitudinal line through the centre portion of the elasticated area and parallel to the longitudinal axis of the topsheet, said angle being between 10° and 50°, or preferably between 17° to 45°, or even more preferably between 25° and 45°.

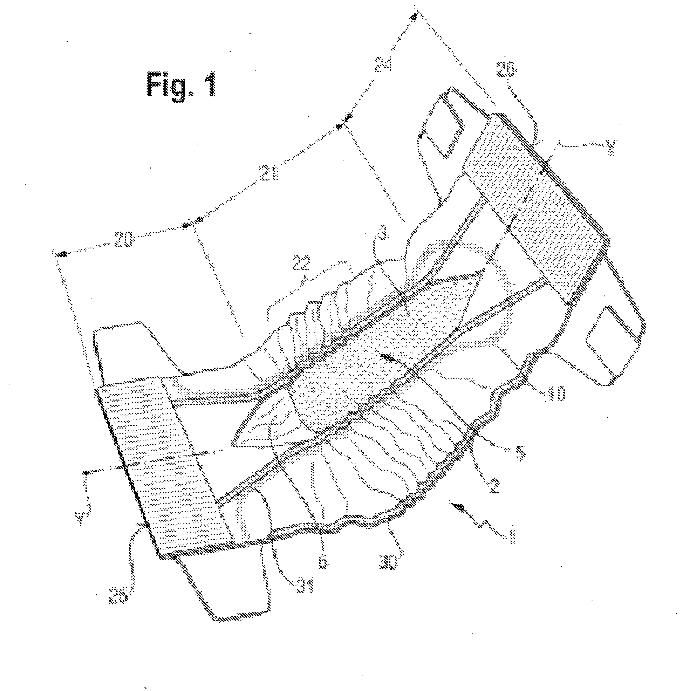

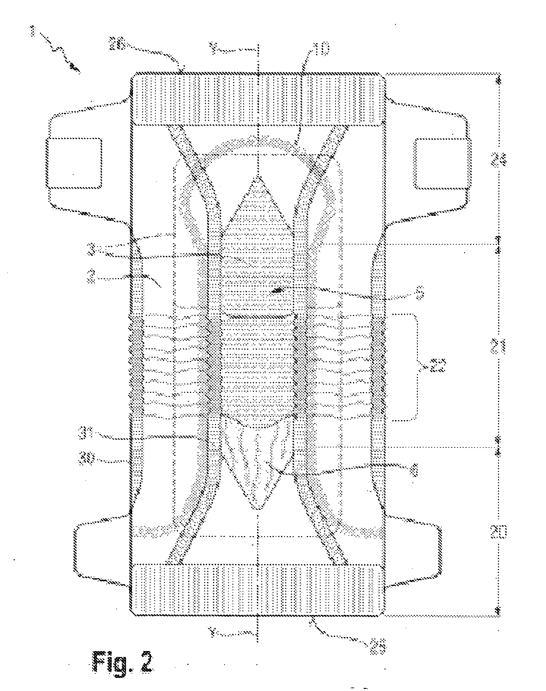

When both front end portions and both back end portions have an angle as above, then the elasticated areas have, as is herein referred to, an X-shape, and a preferred X-shape is exemplified in Figures 1 and 2.

The topsheet may have a crotch area (21), being the centre 30% of the topsheet, in longitudinal direction, and it may then comprise a secondary elasticated areas in said crotch area, for example on either longitudinal side of the opening(s) or part thereof, typically extending in longitudinal direction between a longitudinal side edge of the topsheet and the elasticated area (described above) closest to said edge. Such a secondary elasticated area may have an overall curvature, curving away from the closest elasticated area, described above.

Preferably, the (secondary) elasticated areas herein are formed by attaching an elasticated material in stretched state or partially being in stretched state to part of the topsheet material, e.g. the nonwoven sheet described herein, or to one or more carrier material(s), which is (are) then subsequently attached to part of the topsheet or nonwoven sheet. In one embodiment, the elastic material is attached to the nonwoven sheet material described herein on the surface area of the sheet that is not in contact with the skin of the user; and/ or it is attached to the nonwoven sheet with one a first surface area of the elastic material and a protective sheet material is attached to the opposite surface area of the elastic material; and/ or it is attached to said nonwoven sheet material with a first surface area of the elastic material and then said nonwoven sheet material is subsequently folded, e.g. in a C-fold shape, over the opposite side of the elastic material, to form the elasticated area.

The longitudinal side edges of the topsheet are preferably joined or attached to the longitudinal side edges of the backsheet, by any attachment means known in the art, to form longitudinal opposing attachment areas. In one preferred embodiment of the present invention, the topsheet and the backsheet are attached directly to one another in some locations and are indirectly joined together in other locations.

Preferably, the absorbent article of the invention is sag-tolerable, and it thereto has preferably a topsheet that is sag-tolerable as defiend and described in EP1279388-A. This means that the topsheet does not sag and that the topsheet keeps its z-direction alignment with the anal region and genitals of the wearer, and typically also its x and y direction alignment, when the backsheet and absorbent core sag due to increased weight of the body exudates received by the article. The absorbent article, preferably diaper or training pants, has thereto means to ensure that the topsheet stays in about the same contact or close proximity with the wearer's anal and/ or genital region when the backsheet and core sag, compared to just after application of the article to the wearer, when the backsheet and core do not yet sag. Typically the topsheet is sag-tolerable to such an extend that when the geometrical centre point of the backsheet is pulled down with 4 cm, the topsheet does not move down with more than 0.5 cm, or even not more than 0.25 cm, or typically the topsheet does not move down at all, and/ or such that the longitudinal side edges of the opening do not move in the x and y direction with more than 0.5 cm, or preferably not more than 0.25 cm, or does not move at all.

In one embodiment, the absorbent article has a transverse centre line defining the crotch point on the edges of the absorbent core and the crotch points on the longitudinal edges of the opening in the topsheet herein, and the width from the crotch point on a longitudinal edge to the crotch point on the core edge is preferably at least 50% of the average width of the core, and/ or at least 50% of the width of the core in the crotch line (transverse centre line), or preferably at least 60% or at least 70% or at least 80%.

Preferably, the average width of the topsheet, including the width of the opening, is larger than the average distance between the longitudinal attachment areas of the topsheet to the backsheet, mentioned above. Also preferred is that the average width of the topsheet, including the width of the opening, is larger than the average width of the backsheet. The topsheet may for example have one or more transverse and/ or, more preferably, longitudinal folds, which can unfold in use and allow sagging of the core and backsheet, while the topsheet remains in place.

The topsheet herein may be liquid or urine pervious or impervious. It may be preferred that the topsheet is liquid or urine pervious in one direction, but liquid or urine impervious in the opposite direction, e.g. that body fluids may penetrate through the topsheet to the remaining part of the diaper, but that no or limited amounts of liquid (urine) can penetrate in reverse direction, towards the wearer's skin.

However, in a highly preferred embodiment, the topsheet or at least more than 50% of its surface area (that faces the wearer in use) is hydrophobic. The topsheet may be substantially urine impermeable and feces impermeable.

Preferred topsheets herein are considered urine-impermeable and feces impermeable and thus suitable herein, when they have a low surface energy and a uniform pore size distribution, preferably with the low surface energy values, pore sizes and air permeability values described in co-pending application EP-A-1417945. Preferred substantially impermeable materials with an alcohol repellency of at least 5 or at least 6 or at least 7, or at least 8; preferably having a surface energy of between 20 and 35 mN/m; optionally having a contact angle with water of above 100°; and optionally having a mean pore size of less than 50 microns, preferably less than 30 microns, or less than 20 microns, but optionally at least 2 microns, or at least 5 microns. Preferably, the topsheet may have an air permeability of at least 3 Darcy, or at least 10 Darcy, or at least 20 Darcy, or at least 30 Darcy.

As mentioned above, preferred topsheets herein are made of hydrophobic material or are treated to be hydrophobic (in order to isolate the wearer's skin from liquids contained in remaining part of the diaper), with for example a hydrophobic surface coating. Preferred hydrophobic surface coatings or barrier agent/ masking facilitating agents are for example described in co-pending application US60/543,785, filed February 11, 2004. Preferably, the hydrophobic surface coating or masking facilitating agent comprises one or more silicone polymers or fluorinated polymers. Suitable silicone polymers are for example selected from the group consisting of silicone MQ resins, polydimethysiloxanes, crosslinked silicones, silicone liquid elastomers, and combinations thereof. Typically, the molecular weight of such silicone polymers should be at least about 4000 MW, preferably at least about 10,000 MW, more preferably at least about 15,000 MW, even more preferably at least about 20,000 MW, and most preferably at least about 25,000 MW. Preferred polydimethylsiloxanes are selected from the group consisting of vinyl-terminated polydimethsiloxanes, methyl hydrogen dimethylsiloxanes, hydroxyl-terminated polydimethysiloxanes, organo-modified polydimethylsiloxanes, and combinations thereof. Suitable fluorinated polymers are selected from the group consisting of telomers and polymers containing tetrafluoroethylene and/or perfluorinated alkyl chains. For instance, fluorinated surfactants, which are commercially available from Dupont under the tradename Zonyl®, are suitable for use herein. In particular, Zonyl® 321, 329, 8740, 9027, and 9360 are well suited for use in the present invention. Additionally, other Zonyl® materials include fluroadditives like micro-powders may be useful herein. These include, but are not limited to Zonyl® MP1100, MP1200, MP1400, MP1500J, MP1600N, TE-3667N (which is a water dispersion). Preferably, the coating is free of aminosilicones.

These materials are preferably deposited onto the topsheet in amounts of from at least about 0.01 gsm (gram of material/square meter of topsheet), more preferably from at least about 0.05 gsm, and most preferably from at least about 0.1gsm.

Any portion of the topsheet may be coated with a lotion or powder, known in the art. Preferred may be that at least lotion is present on the primary elasticated areas, and even preferably on the secondary elasticated areas. The lotion used on one elasticated area may be different to the lotion used on another elasticated area, or on the remaining part of the topsheet. Examples of lotions include those described in U.S. 5,607,760; U.S. 5,609,587; U.S. 5,635,191; U.S. 5,643,588; WO 95/24173, provided the lotion is compatible with the elastic material, and does not destroy the elastic material or reduce its elasticity.

The absorbent article also comprises a, typically liquid impervious, backsheet, as known in the art. In preferred embodiments, the liquid impervious backsheet comprises a thin plastic film such as a thermoplastic film having a thickness of about 0.01 mm to about 0.05 mm. Suitable backsheet materials comprise typically breathable material, which permit vapors to escape from the diaper while still preventing exudates from passing through the backsheet. Suitable backsheet films include those manufactured by Tredegar Industries Inc. of Terre Haute, IN and sold under the trade names X15306, X10962 and X10964.

The backsheet, or any portion thereof, may be elastically extendable in one or more directions.

The backsheet may be attached or joined to the topsheet, the absorbent core, or any other element of the diaper by any attachment means known in the art. It may be highly preferred that the longitudinal side edges of the topsheet and backsheet are directly attached to one another, but that the longitudinal edges of the topsheet and the core are not attached to one another.

The attachment means to attach the topsheet and the backsheet, but also the genital coversheet herein may include a uniform continuous layer of adhesive, a patterned layer of adhesive, or an array of separate lines, spirals, or spots of adhesive, such as disclosed in U.S.4,573,986. Adhesives that have been found to be satisfactory are manufactured by H. B. Fuller Company of St. Paul, Minnesota and marketed as HL-1620 and HL-1358-XZP. Alternatively, the attachment means may comprise heat bonds, pressure bonds, ultrasonic bonds, dynamic mechanical bonds, or any other suitable attachment means or combinations of these attachment means as are known in the art.

The absorbent core may comprise any absorbent material which is generally compressible, conformable, non-irritating to the wearer's skin, and capable of absorbing and retaining urine, such as comminuted wood pulp, creped cellulose wadding; melt blown polymers, including coform; chemically stiffened, modified or cross-linked cellulosic fibers; tissue, including tissue wraps and tissue laminates; absorbent foams; absorbent sponges; super absorbent polymers; absorbent gelling materials; or any other known absorbent material or combinations of materials; preferred may be absorbent cores which have an absorbent storage layer which comprises more than 80% by weight of the absorbent core content (e.g. excluding core wrap) of absorbent gelling material, and which is preferably free of airfelt.

The absorbent article may also include a sub-layer (which may be the same as the body facing liner described above) disposed between the topsheet and the absorbent core, capable of accepting, and/ or immobilizing bodily exudates, typically fecal material. Suitable materials for use as the sub-layer may include large cell open foams, macro-porous compression resistant non woven highlofts, large size particulate forms of open and closed cell foams (macro and/or microporous), highloft non-wovens, polyolefin, polystyrene, polyurethane foams or particles, structures comprising a multiplicity of vertically oriented, preferably looped, strands of fibers, or preferably apertured formed films, as described above with respect to the genital coversheet. (As used herein, the term "microporous" refers to materials that are capable of transporting fluids by capillary action, but having a mean pore size of more than 50 microns. The term "macroporous" refers to materials having pores too large to effect capillary transport of fluid, generally having pores greater than about 0.5 mm (mean) in diameter and more specifically, having pores greater than about 1.0 mm (mean) in diameter, but typically less than 10 mm or even less than 6 mm (mean). The absorbent article herein is preferably a disposable adult or infant diaper or training pants/ pull-up pants. The diapers herein preferably have a fastening system, typically joined to the waistband, as known in the art. Preferred fastening systems comprise fastening tabs and landing zones, wherein the fastening tabs are attached or joined to the back region of the diaper and the landing zones are part of the front region of the diaper.

Preferred may be that the articles of the invention (e.g. diaper) when packed in their packaging material, comprise two transverse folds, so that when unfolded for use by the user or care taker, the article (e.g. diaper) is in a U-shape and easier to apply.

### Test methods

### Handle-o-meter bending rigidity test

This method serves to determine the bending rigidity (and thereby softness) of a nonwoven layer or nonwoven sheet, as described herein, and reflects the flexibility and surface friction of the material. In this test, a nonwoven is deformed through a slot by use of a.plunger, and the required force is measured. This method is based on the INDA Standard test IST 90.3-92

A sample material of the nonwoven sheet or nonwoven layer of 1 inch long and 1 inch wide (25mm x 25mm) is cut and conditioned at 65% humidity and 21°C as set out in the INDA test. The sample is free form elastic material or edges attached to other materials. In one embodiment, the average fiber direction of the nonwoven web or layer in contact with the skin in use can be determined and this would be the Y direction (e.g. in use typically corresponding MD dimension of the absorbent article). A handle-o-meter, available from Twingh- Albert Instruments Co., Philadelphia, USA, is calibrated as set out its user instructions.

The slot width is 6.35mm.

The sample is placed under the plunger and on the slot with the surface that in use contacts or faces the skin up wards facing up. A first dimension is perpendicular to the slot and this is the direction tested, for which the bending rigidity is reported herein. In one embodiment, this is the average fiber direction of the skin-facing surface, e.g. the spunbond layer. The sample is centered over the slot and the test is run and the force is measured. This value is *multiplied by 4* (e.g. normalised to a 4 inch x 4 inch sample) and reported in grams herein as the bending rigidity.

### Hydrostatic head (hydrohead)

The hydrostatic head (also referred to as hydrohead) as used herein is measured with a low surface tension liquid, i.e. a 49 mN/m liquid (solution).

This liquid is prepared as set out below.

This test is performed as set out in co-pending application WO2005/112854A, conform the Inda/ Edana test WSP 80.6 (05). However, the water pressure (from below) is increased with a rate is 60 mbar/min.

A sample of 5 cm² is taken from the nonwoven sheet or cuff or topsheet herein. The sample should be free from elastic material or edges that are connected to other materials.

The test head used has a 2.5 cm diameter; the protective sleeve used has a 2.2 cm diameter.

The test is performed on this sample and the Hydrostatic head value is obtained, and referred to herein.

### 49 mN/m (dynes/cm) liquid preparation:

A 10 litre canister with tap_is cleaned thoroughly 3 times with 2 litres polyethylene and then 3 times with 2 litres distilled/deionized water.

Then, it is filled with 10 litres distilled/deionized water and stirred with a clean stirring bar for 2 h, after which the water is released via the tap.

A 5 litre glass is cleaned 6 times with water and then 6 times with distilled/deionized water.

Then, 30.00 g of Na Cholate and 5 litres of distilled/deionized water are placed in the cleaned 5 litres glass. (NaCholate should have a TLC purity of >99%, e.g. supplied by Calbiochem, catalog # 229101). This is stirred with a clean stirring bar for about 5 min, until the Na Cholate is visibly dissolved.

The stirring bar is removed from the glass with a magnetic stick (without touching the solution) and then the Na cholate solution is poured into the 10 litres canister and more distilled/deionized water is added such that the concentration of the final solution is 3 g/l. This is further stirred with a stirring bar for 2 hours and then used. This preparation of the solution and use thereof is at the temperature stated for the test for which it is used, or if no temperature is stated, it is kept at 20°C.

The surface tension of the solution is measured and this should be 49 mN/m (+/- 2). (The surface tension may be determined by method: ASTM D1331-56 ("Standard test method for surface and interfacial tension of solution of surface active agents") using a Kruss K12 tensiometer.)

### Low surface tension Strike Through value method

The low surface tension strike through value referred to herein may be obtained by the Edana method WSP70.3 (05), except that a low surface tension liquid (see below) is used and a sample of 1 inch x 1 inch (25 mm x 25 mm) may be used. The sample should be free of elastic material or of edges that are connected to other materials.

The value obtained from this sample measurement is reported herein.

The low surface tension liquid is a liquid with a surface tension of 32 mN/m prepared as follows:

In a clean flask, 2.100 grams of Triton-X-100 is added to 500 ml distilled water (already in flask) and then 5000 ml distilled water is added. The solution is mixed for 30 minutes and then the surface tension is measured, which should be 32 mN/m. (The surface tension may be determined by method: ASTM D1331-56 ("Standard test method for surface and interfacial tension of solution of surface active agents") using a Kruss K12 tensiometer.)

While particular embodiments of the present invention have been illustrated and described, it would be obvious to those skilled in the art that various other changes and modifications that are within the scope of this invention. Each embodiment defined by certain properties or dimension for which a value is defined herein is to be understood to include embodiments with functional equivalent properties or dimensions, e.g. a dimension of 0.5 cm has to be understood as meaning "about 0.5 cm".

## Claims

1. An absorbent article comprising a backsheet, absorbent core and topsheet, said topsheet having at least one opening to receive feces and said topsheet comprising a nonwoven sheet, whereby said nonwoven sheet comprises at least two nonwoven layers that are not fully attached to one another, preferably having an attachment area of 60% or less, each comprising two or more nonwoven webs that are laminated to one another, and whereby the nonwoven sheet comprises nano-fibers and/ or meltblown fibers, said meltblown fibers preferably being present at a level of at least 5 g/m², and the nonwoven sheet having a basis weight of 40 g/m² or less, or preferably 30 g/m² or less.

2. An absorbent article as in claim 1, whereby said nonwoven layers have an attachment area of 40% or less.

3. An absorbent article as claim 1 or 2, whereby said nonwoven sheet comprises two or more nonwoven layers that comprise each two or more nonwoven webs, whereby each nonwoven layer comprises meltblown fibers and/ or nano-fibers, and said nonwoven sheet has a basis weight of less than 45 g/m² or preferably 30 g/m² or less, and/ or the nonwoven layer has a basis weight of 25 g/m² or less.

4. An absorbent article as in claim 1, whereby each nonwoven layer comprises at least 5g/m² meltblown fibers and/ or at least 2 g/m³ nano-fibers.

5. An absorbent article as in any preceding claim, whereby said nonwoven sheet has a hydrostatic head of at least 30 mbar.

6. An absorbent article as in any preceding claim, whereby said skin-facing nonwoven layer or web has a bending rigidity of 14 grams or less, and/ or whereby said nonwoven sheet has a bending rigidity of less than 25 grams.

7. An absorbent article as in any preceding claim, whereby the skin-facing nonwoven web or the skin-facing nonwoven web comprised by said skin-facing nonwoven layer is a spunbond web, comprises fibers with an average fiber direction and said web having a bending rigidity in said fiber direction of 20 grams or less, preferably 16 grams or les, or 14 grams or less.

8. An absorbent article as in any preceding claim, whereby said nonwoven sheet has a low surface tension strike through value of more than 55 seconds.

9. An absorbent article as in any preceding claim, whereby said topsheet has an opening in the form of a slit opening with longitudinally opposing side edges and comprising at least a pair of elastic components, whereof at least one elastic component is present along or in close proximity to each longitudinally extending side edge.

10. An absorbent article as in any preceding claim, whereby said nonwoven sheet comprises a coating of a barrier agent, selected from silicone polymers or fluorinated polymers.

## Patentansprüche

1. Absorptionsartikel mit einer Unterschicht, einem Absorptionskern und einer Oberschicht, wobei die Oberschicht mindestens eine Öffnung für die Aufnahme von Fäkalien aufweist und die Oberschicht eine Vlieslage umfasst, wobei die Vlieslage mindestens zwei Vliesschichten aufweist, die nicht vollständig miteinander verbunden sind, vorzugsweise mit einem Bindungsbereich von 60 % oder weniger, wobei jede zwei oder mehr Vliesbahnen umfasst, die aneinander laminiert sind, und wobei die Vlieslage Nanofasern und/ oder schmelzgeblasene Fasern umfasst, wobei die schmelzgeblasenen Fasern vorzugsweise in einer Konzentration von mindestens 5 g/m² vorhanden sind und die Vlieslage ein Basisgewicht von 40 g/m² oder weniger oder vorzugsweise 30 g/m² oder weniger aufweist.

2. Absorptionsartikel nach Anspruch 1, wobei die Vliesschichten einen Bindungsbereich von 40 % oder weniger aufweisen.

3. Absorptionsartikel nach Anspruch 1 oder 2, wobei die Vlieslage zwei oder mehr Vliesschichten umfasst, die jeweils zwei oder mehr Vliesbahnen umfassen, wobei jede Vliesschicht schmelzgeblasene Fasern und/oder Nanofasern umfasst und die Vlieslage ein Basisgewicht von weniger als 45 g/m² oder vorzugsweise 30 g/m² oder weniger aufweist und/oder die Vliesschicht ein Basisgewicht von 25 g/m² oder weniger aufweist.

4. Absorptionsartikel nach Anspruch 1, wobei jede Vliesschicht mindestens 5 g/m² schmelzgeblasene Fasern und/oder mindestens 2 g/m³ Nanofasern umfasst.

5. Absorptionsartikel nach einem der vorgenannten Ansprüche, wobei die Vlieslage einen hydrostatischen Druck von mindestens 30 mbar aufweist.

6. Absorptionsartikel nach einem der vorgenannten Ansprüche, wobei die hautseitige Vliesschicht bzw. die hautseitige Bahn eine Biegesteifigkeit von 14 Gramm oder weniger aufweist, und/oder wobei die Vlieslage eine Biegesteifigkeit von weniger als 25 Gramm aufweist.

7. Absorptionsartikel nach einem der vorgenannten Ansprüche, wobei die hautseitige Vliesbahn oder die hautseitige Vliesbahn, die in der hautseitigen Vliesschicht enthalten ist, eine Spinnvliesbahn ist, Fasern mit einer durchschnittlichen Faserrichtung umfasst und die Bahn eine Biegesteifigkeit in Faserrichtung von 20 Gramm oder weniger aufweist, vorzugsweise 16 Gramm oder weniger oder 14 Gramm oder weniger.

8. Absorptionsartikel nach einem der vorgenannten Ansprüche, wobei die Vlieslage einen niedrigen Oberflächenspannungs-Durchschlagwert von mehr als 55 Sekunden aufweist.

9. Absorptionsartikel nach einem der vorgenannten Ansprüche, wobei die Oberschicht eine Öffnung in Form einer Spaltöffnung mit einander längs gegenüberliegenden Seitenrändern aufweist und mindestens ein Paar elastische Komponenten enthält, wovon mindestens eine elastische Komponente entlang oder in unmittelbarer Nähe zu jedem längs verlaufenden Seitenrand vorliegt.

10. Absorptionsartikel nach einem der vorgenannten Ansprüche, wobei die Vlieslage eine Beschichtung mit einem Sperrmittel ausgewählt aus Siliconpolymeren oder fluorierten Polymeren umfasst.

## Revendications

1. Article absorbant comprenant une feuille de fond, une âme absorbante et une feuille de dessus, ladite feuille de dessus ayant au moins une ouverture pour recevoir les matières fécales et ladite feuille de dessus comprenant une feuille non tissée, selon lequel ladite feuille non tissée comprend au moins deux couches de non-tissé qui ne sont pas complètement fixées l'une à l'autre, ayant de préférence une zone d'attachement de 60 % ou moins, comprenant chacune deux ou plusieurs nappes non tissées qui sont stratifiées l'une à l'autre, et selon lequel la feuille non tissée comprend des nanofibres et/ou des fibres soufflées en fusion, lesdites fibres soufflées en fusion étant de préférence présentes à un taux d'au moins 5 g/m², et la feuille non tissée ayant une masse surfacique de 40 g/m² ou moins, ou de préférence 30 g/m² ou moins.

2. Article absorbant selon la revendication 1, selon lequel lesdites couches de non-tissé ont une zone d'attachement de 40 % ou moins.

3. Article absorbant selon la revendication 1 ou 2, selon lequel ladite feuille non tissée comprend deux ou plusieurs couches de non-tissé qui comprennent chacune deux ou plusieurs nappes non tissées, selon lequel chaque couche de non-tissé comprend des fibres soufflées en fusion et/ou des nanofibres, et ladite feuille non tissée a une masse surfacique de moins de 45 g/m² ou de préférence 30 g/m² ou moins, et/ou la couche de non-tissé a une masse surfacique de 25 g/m² ou moins.

4. Article absorbant selon la revendication 1, selon lequel chaque couche de non-tissé comprend au moins 5 g/m² de fibres soufflées en fusion et/ou au moins 2 g/m³ de nanofibres.

5. Article absorbant selon l'une quelconque des revendications précédentes, selon lequel ladite feuille non tissée a une charge hydrostatique d'au moins 30 mbar.

6. Article absorbant selon l'une quelconque des revendications précédentes, selon lequel ladite couche ou nappe de non-tissé faisant face à la peau a une rigidité au pliage de 14 grammes ou moins, et/ou selon lequel ladite feuille non tissée a une rigidité au pliage de moins de 25 grammes.

7. Article absorbant selon l'une quelconque des revendications précédentes, selon lequel la nappe non tissée faisant face à la peau ou la nappe non tissée faisant face à la peau comprise par ladite couche de non-tissé faisant face à la peau est une nappe filée-liée, comprend des fibres avec une direction moyenne de fibre et ladite nappe ayant une rigidité au pliage dans ladite direction de fibre de 20 grammes ou moins, de préférence 16 grammes ou moins, ou 14 grammes ou moins.

8. Article absorbant selon l'une quelconque des revendications précédentes, selon lequel ladite feuille non tissée a une valeur de pénétration de faible tension superficielle de plus de 55 secondes.

9. Article absorbant selon l'une quelconque des revendications précédentes, selon lequel ladite feuille de dessus a une ouverture sous la forme d'une ouverture de fente avec des bords latéraux longitudinalement opposés et comprenant au moins une paire de composants élastiques, desquels au moins un composant élastique est présent le long de ou à proximité immédiate de chaque bord latéral s'étendant longitudinalement.

10. Article absorbant selon l'une quelconque des revendications précédentes, selon lequel ladite feuille non tissée comprend un revêtement d'un agent de barrière, choisi parmi les polymères de silicone ou les polymères fluorés.
